(19) Europäisches Patentamt
European Patent Office
Office européen des brevets

(11) Publication number: **0 102 554**
**B1**

(12) **EUROPEAN PATENT SPECIFICATION**

(45) Date of publication of patent specification: **29.04.87**

(51) Int. Cl.[4]: **A 61 K 31/265,**
C 07 C 153/11, C 07 D 295/08

(21) Application number: **83107891.0**

(22) Date of filing: **10.08.83**

(54) **Compounds for dilating the smooth muscles of the upper urinary tract.**

(30) Priority: **27.08.82 US 412238**

(43) Date of publication of application:
**14.03.84 Bulletin 84/11**

(45) Publication of the grant of the patent:
**29.04.87 Bulletin 87/18**

(84) Designated Contracting States:
**BE CH DE FR GB IT LI LU NL SE**

(56) References cited:
**US-A-2 390 555**

**CHEMICAL ABSTRACTS, vol. 61, no. 1, 6th July 1964, abstract no. 1141f-g, Columbus, Ohio, US. YU. V.NATOCHIN et al.: "The search for pharmacological agents capable of depressing the antidiuretic hormone"**

**CHEMICAL ABSTRACTS, vol. 92, no. 13, 31st March 1980, page 72, no. 104526s, Columbus, Ohio, US. V. G. KUKES et al.: "Data on the efficacy of tiphen"**

(73) Proprietor: **United Pharmaceuticals, Inc.**
**1500 North Wilmot Road**
**Tucson Arizona 85712 (US)**

(72) Inventor: **Davis, William M**
**3501 N. Bear Canyon Road**
**Tuscon Arizona 85712 (US)**

(74) Representative: **Weber, Dieter, Dr. et al**
**Dr. Dieter Weber und Klaus Seiffert**
**Patentanwälte Gustav-Freytag-Strasse 25**
**Postfach 6145**
**D-6200 Wiesbaden 1 (DE)**

(56) References cited:
**L. P. THACKSTON, The Journal of Urology, 73(3), 1955, pp 487-493**

**The Ureter, Bergman, 1st Ed., 1967, pp 158-177**
**O'REILLY, P. H. ed. Idiopathic Hydrorephrosis, Berlin 1982, pp 4-8**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 102 554 B1**

**Description**

This invention relates to the use of di-N-substituted aminoethyl ester of diphenylthioacetic acid for the preparation of a drug for the dilation of the smooth muscles of the upper urinary tract. Such an invention has many novel applications including but not limited to the treatment of patients passing kidney stones through the upper urinary tract.

To date, no clinically effective upper urinary tract dilator is in use. Currently used urinary tract dilators are generally anti-cholinergic drugs, such as papaverine, a narcotic derivative. These drugs have either been ineffective as dilators or have undesirable side effects such as dryness of the mouth, blurred vision, and slowed heart beat.

The di-N-substituted aminoethyl esters of diphenylthioacetic acid have the formula:

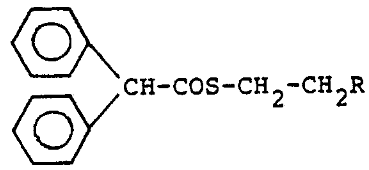

in which R represents a diethylamino group, a morpholino group or a piperidino group.

Thiphenamil hydrochloride is a well-known compound of the above formula wherein R represents a diethylamino group and is described in detail in the US—A—2 390 555. Additionally methods of making thiphenamil hydrochloride are described in the US—A—2 510 773.

From Chemical Abstracts 92 (1980), page 72, No. 104 526 s, it is known, that the thiphenamil hydrochloride has a spasmolytic activity on the gastro-intestinal tract and the bladder similar to that of papaverine. But typical direct smooth muscle relaxing drugs, such as papaverine, have not been shown to significantly effect the ureter and the renal pelvis (upper urinary tract). Moreover, it is known from "Idiopathic Hydronephrosis", edited by P. H. O'Reilly and J. A. Gosling, Springer-Verlag, 1982, pages 4 to 8, and from Bergman "The Ureter", Springer-Verlag, 2nd edition, 1982, Chapter 6, pages 140 to 146, that the mechanism of innervation of the gastro-intestinal tract and the bladder is completely different from that of the upper urinary tract. There is a heavy autonomic nervous and cholinergic innervation of the gastro-intestinal tract and of the bladder, but no cholinergic and exceedingly sparse autonomic nervous innervation of the upper urinary tract. The contraction of the renal pelvis and ureter originates from an unique pacemaker mechanism located in the smooth muscles of the minor calices of the renal pelvis. Thus, one could not expect a drug found to be useful on the gastro-intestinal tract or bladder to be effective on the upper urinary tract. L. P. Thackston et al, in the Journal of Urology, 73(3), pp. 487—493 have described a method of treatment of spastic ureteritis involving ureteral catheters wherein β-diethylaminoethyl diphenylthioacetate is used as an adjunct.

An important object of the present invention is to provide a means for dilating and relaxing the smooth muscles of the upper urinary tract without subjecting the patient to the undesirable side effects of anti-cholinergic drugs, for example dryness of the mouth, dilation of the pupils, and slowed heart beat.

It is a further important object of the present invention to provide such a means which is safe for use on human patients and which is also safe for particularly sensitive patients such as glaucoma patients.

It is yet another object of the present invention to provide a means for treating patients passing kidney stones without subjecting the patient to surgery.

It is another important object of the present invention to provide such a means of dilating and relaxing the smooth muscles of the upper urinary tract without leaving or accumulating any foreign substances in the human body tissue.

These and other objects are met by the use of a di-N-substituted aminoethyl ester of diphenylthioacetic acid having the formula:

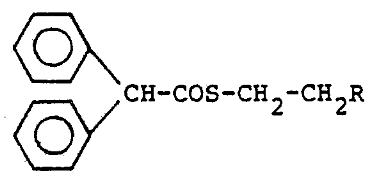

in which R represents a diethylamino group, a morpholino group or a piperidino group, or a hydrochloride thereof for the preparation of a drug for the dilation of the smooth muscles of the upper urinary tract.

Such a drug is especially used to treat a patient passing a kidney stone.

It is surprising that the hydrochlorides of the above formula can be used for dilating that smooth muscles of the upper urinary tract and specifically to dilate and relax the smooth muscles of the upper urinary tract in order to treat a patient passing a kidney stone through the upper urinary tract, in the absence of any surgical intervention.

It is evident from the experimental results which follow that the hydrochlorides of compounds of the above formula increase the compliance of the renal pelvis. Under conditions of increased compliance, the capacity of the renal pelvis is significantly increased. Additionally, the renal pelvis pressure is concomitantly reduced with the contractile rate. Under these circumstances, the upper urinary tract dilates and peristaltic contractions are greatly diminished in both amplitude and frequency.

The experimental results reveal that the hydrochlorides of compounds of the above formula act as effective upper urinary tract dilators in a dosage range of from 0.7 to 11.4 mg per kilogram of body weight. A preferred dosage is in the range of from 1.4 to 5.7 mg per kilogram of body weight. A still more preferred dosage range is from 2.8 to 5.7 mg per kilogram of body weight.

The hydrochlorides of compounds of the above formula can be administered orally, typically in tablets of 100—400 mg, or by intravenous injection.

Because the hydrochlorides of the compounds of the above formula slowly hydrolyze in water, they are generally not used as a serum or suspension. It is possible, however, to encapsulate microspheres of the hydrochlorides in the form of liquid suspensions for administration to patient.

The invention is further disclosed by means of the following examples which are intended only as illustrations and which is no way to limit the invention.

Example 1

Nine pigs, five male and four female, were implanted with a telemetry package to record the amplitude and frequency of the filling and emptying phase of the renal pelvis. The pigs were lightly anesthetized with 2% halothane and 98% oxygen. A dose of 1 milligram of thiphenamil hydrochloride (in which R is a diethylamino group) per kilogram of body weight (mg/kg) was given to each pig intravenously through an ear vein following a 20 minute control period.

The same procedures were repeated using a dose of 1 milligram of the hydrochloride of a compound of the above formula in which R is a morpholino group per kilogram of body weight (mg/kg).

The same procedures were repeated using a dose of 1 milligram of the hydrochloride of a compound of the above formula in which R is a piperidino group per kilogram of body weight (mg/kg).

The dose of 1 mg/kg of all three hydrochlorides increased the rate of contraction in the renal pelvis from $1.84 \pm 0.15$ to $2.03 \pm 0.35$ contractions per minute.

Example 2

The same procedure as utilized in Example 1 was used on the nine pigs which were given a dose of 10 milligrams of: first, thiphenamil hydrochloride per kilogram of body weight (mg/kg); second, a 10 milligram dose of the hydrochloride of a compound of the above formula in which R is a morpholino group per kilogram of body weight (mg/kg); and third, a 10 milligram dose of the hydrochloride of a compound of the above formula in which R is a piperidino group per kilogram of body weight (mg/kg).

The in vivo data show that the effect of the above hydrochlorides on renal pelvic frequency is bimodal. Thus, while at a dose of 1 mg/kg of all three hydrochlorides the renal pelvis contraction frequency increases, at doses greater than 1 mg/kg of the three hydrochlorides the frequency of pelvic contractions decreases significantly. The data follows a roughly linear equation computed from the dose response curves:

$$\Delta F = -11.5 - 3.3X$$

where $\Delta F$ is the percent change in frequency and X is the dose of the hydrochloride in milligrams per kilogram of body weight.

In the test animals, renal pelvic pressure decreased significantly from a means of $647{,}5 \pm 64{,}8$ Pa to $225{,}6 \pm 15{,}7$ Pa.

In five of the experimental animals, measures were taken with the abdomen open and the ureter obstructed. Under these circumstances renal pelvic pressure was effectively raised to a mean value of $1805{,}0 \pm 618{,}0$ Pa. Intravenous thiphenamin hydrochloride at 2.5 mg/kg significantly reduced the resting renal pelvic pressure to $578{,}8 \pm 304{,}1$ Pa in four of the five test animals.

The data also demonstrate a linear decrease in the frequency of spontaneous renal pelvic contractions. A linear equation computed from the dose response curves is given by:

$$\Delta F = -5.2 - 5.6X$$

where $\Delta F$ is the percent change in frequency and X is the dose of the hydrochloride in 10—3M. Furthermore, the amplitude of the spontaneous contractions decreased according to the relationship:

$$\Delta T = 1.2 + 0.4X$$

where $\Delta T$ is the percentage decrease in amplitude of contraction and X is the dose of the hydrochloride at 10—3M.

## Claim

Use of a di-N-substituted aminoethyl ester of diphenylthioacetic acid having theformula:

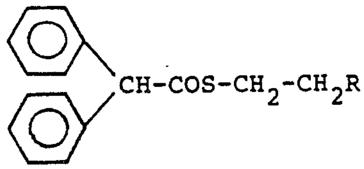

in which R represents a diethylamino group, a morpholino group or a piperidino group, or a hydrochloride thereof for the preparation of a drug for the dilation of the smooth muscles of the upper urinary tract.

## Patentanspruch

Verwendung eines di-N-substituierten Aminoethylesters von Diphenylthioessigsäure mit der Formel

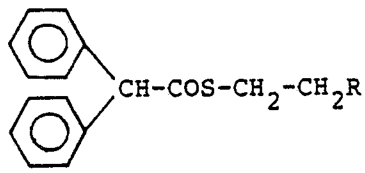

worin R eine Diethylaminogruppe, eine Morpholinogruppe oder eine Piperidinogruppe bedeutet, oder eines Hydrochlorids desselben für die Herstellung eines Arzneimittels für die Erweiterung der glatten Muskulatur der oberen Harnwege.

## Revendication

Emploi d'un ester aminoéthylique d'acide diphénylthioacétique di-N-substitué répondant à la formule:

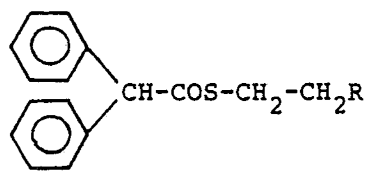

dans laquelle R représente un groupe diéthylamino, un groupe morpholino ou un group pipéridino, ou d'un de ses chlorhydrates, pour la préparation d'un médicament pour la dilatation des muscles lisses des voies urinaires supérieures.